**Europäisches Patentamt**

**European Patent Office**

**Office européen des brevets**

(19)

(11) Publication number: **0 057 963**

A2

(12) # EUROPEAN PATENT APPLICATION

(21) Application number: **82200146.7**

(22) Date of filing: **05.02.82**

(51) Int. Cl.³: **C 10 L 1/00**

(30) Priority: **07.02.81 DE 3104334**
**21.01.82 DE 3201739**

(43) Date of publication of application:
**18.08.82 Bulletin 82/33**

(84) Designated Contracting States:
**AT BE CH DE FR GB IT LI LU NL SE**

(71) Applicant: **Pruss, Günter**
**Tilsiter Strasse 2 L**
**D-2217 Kellinghusen/Holstein(DE)**

(72) Inventor: **Pruss, Günter**
**Tilsiter Strasse 2 L**
**D-2217 Kellinghusen/Holstein(DE)**

(74) Representative: **Huygens, Arthur Victor, Dr. et al,**
**c/o Gist-Brocades N.V. Patent & Trademarks**
**Department Wateringseweg 1 PO-box 1**
**NL-2600 MA Delft(NL)**

(54) **Process for the energetical enrichment of fuel.**

(57) Process for the energetical enrichment of fuel by treating the fuel with biologically active yeast and fuel obtained by this process. A significant decrease of fuel consumption in the operation of combustion engines can be reached by using the energetically enriched fuel of the invention.

EP 0 057 963 A2

Croydon Printing Company Ltd.

Process for the energetical enrichment of fuel.

The invention is relating to a process for the energetical enrichment of fuel.

Due to a steadily increasing oil consumption and to an alarming scarcity of oil the requirement for energy saving measures becomes always severer.

A condition for such measures are alternative solutions, which offer a technical operatability in connection with a possible elegant technology for the improvement of the state of the art.

The most actual measure for diminishing the oil consumption as known is the saving of fuel. This measure is based on a conception to apply for instance methanol as a product derived from brown coal for the application in motor-vehicles. However, such an alternative must be reached by means of expensive coal-liquification processes.

Also other known processes, such as the addition of hydrogen to the normal operation of engines by means of the hydride processes did not appear to be very practical up to now, as the storage elements, which are inevitable, for such processes have to be regarded as rather heavy and therefore as energy consuming.

It is therefore an object of the present invention to avoid the indicated disadvantages with rather simple and less complicated means and to provide a further alternative solution and to improve the yield of the energy conversion by processes of combustion of fuels, as well to make an optimal use of the still present oil reserves.

As a result of research and experimentation it was surprisingly found, that the beforementioned object could be reached by the treatment of fuels like e.g. petrol, gasoil, diesel oil and mixtures thereof with biologically active yeast.

Without any pretension to be able to understand completely the invented effect, this effect might perhaps be

0057963

declared by the assumption that the activity of the biologically active yeast is significantly increased by the consumption of carbon, which is present in the hydrocarbons of the fuel.

It was moreover found that after addition of hydrogen peroxide to the fuel, which was previously treated with the biologically active yeast, before the combustion, an extremely explosive gas mixture is spontaneously created.

Even the reaction of residual yeast with hydrogen peroxide gives rise to an explosive mixture.

Especially violently reacting detonating gas mixtures also are formed after addition of oxygen bearing agents. By all these reactions the formation of hydrogen can be detected.

It will be appreciated that as a result of the present invention a significant decrease of fuel consumption in the operation of combustion engines can be reached.

For example in initial experiments with motor car engines of the injection type, a fuel saving of 0.1-1 and preferably 0.3-0.8 liter pro 100 km could be detected, while moreoever the exhaust gasses appeared to show a very low content of carbon monoxide, relating to a regular and economically improved combustion.

Depending of the type of the engine applied, diminishing of carbonmonoxide in the exhaust gases in an amount of 30-80% by volume could be detected.

Moreover, it could be signalized that the engine, wherein the treated fuel was applied showed a more regular and quiet operation.

The same effects could be detected with application of the pretreated fuels according to the present invention in diesel engines.

It will be appreciated that another feature of the invention is actually formed by fuel compositions obtained by the treatment with a biologically active yeast.

As biologically active yeast may be used as well fresh compressed yeast as dried active yeast, in the form of a powder or of small particles. This treatment of fuel may preferably be carried out in a closed vesselsystem, due to the fermentation like reactions.

According to a specific embodiment of the process of the invention, yeast preparations, industrially prepared by cultivation of strains belonging to the genus Saccharomyces and more particularly Saccharomyces cerivisiae are applied, but also yeasts belonging to e.g. the genus Torulopisis or to other species of Saccharomyces, such as Saccharomyces caarlbergensis, Saccharomyces beticus, Saccharomyces rosei, Saccharomyces cheresiensis, Saccharomyces fermenti and Saccharomyces pasteurianus may be applied with good results.

However, it will be appreciated that preferably yeast preparations are applied, which are manufactured by large scale processes and which are available without any restriction and against relatively low prices in commercial amounts.

It will be appreciated moreover, that also the treatment of fuels with yeast preparations, derived from specific strains adapted to give this special effect in a stronger degree, will be embraced by the scope of the present invention.

According to a preferred embodiment of the process of the present invention, a biologically active dried yeast will be used, enabling a treatment of motor fuels in a rather simple way, e.g. by circulation the petrol through a column filled up with the yeast or simply hanging a filter bag with the yeast in a storage vessel for fuel.

Time ranges for the treatment of fuels with the yeast preparations may vary widely from 5-20 days, depending on practical circumstances e.g. the type of yeast applied. E.g. in the case of applying compressed yeast a treatment of petrol of 5-15 days and preferably 7-10 days at 20 C or more generally room temperature, appeared to give attractive results. In the case of active dried yeast a treatment of petrol of 10-20 days and preferably 12-16 days at roomtemperature has appeared to give attractive results. With longer time ranges autolysis may occur.

- 4 -

0057963

For obtaining the attractive results according to the present invention, the treatment of fuel with 0.01-03 kg. and preferably 0.05-0.15 kg. of yeast, calculated on dry matter pro kg of fuel, consisting of liquid hydrocarbons has appeared to be desirable. It has appeared that the same dried yeast preparations may be used repeatedly e.g. up to four times with good results, after filtrating off the suspended yeast after the fermentation process.

The invention may be illustrated by the following examples, however, without the intention to restrict the scop of the invention in any way.

## Example I.

In an Opel Senator 3,0 E having an injection engine, 15 l. of petrol (quality super) treated with 1 kg of compressed yeast was tested as compared with the same petrol without any treatment. The previous treatment of the petrol with fresh compressed yeast was carried out at 22°C and durin 8 days.

As compared to the untreated super petrol the CO content of the exhaust gases changed from 1% to 0.3% by volume, while the average petrol consumption of 13.5 l. pro 100 km. changed into 12.4 l. pro 100 km.. The exhaustgases obtained when applying the pretreated super petrol, appeared to show a more pleasant odour.

About the same changes could be detected, when super petrol was treated with dried biologically active yeast during 19 days, whereafter no autolysis could be observed.

0057963

## Example II.

In a Volkswagen with a 1.5 l. combustion engine, 15 l. of normal petrol treated with 1 kg. of compressed yeast was tested as compared with the same petrol without any treatment. The previous treatment of the petrol with fresh compressed yeast was carried out during 8 days and at a temperature of 22°C.

As compared to the untreated normal petrol the CO content of the exhaust gases changed from 1.3% to 0.1% by volume, while the average petrol consumption of 10 l./100 km. was changed into 9.2 l./100 km..

When using the previous treated petrol, a better acceleration was obtained, while the engine had better start in the morning. About the same changes could be detected, when normal petrol was treated with dried biologically active yeast during 19 days.

Claims.

**0057963**

1. Process for the energetical enrichment of fuel, characterized in that fuel is treated with biologically active yeast.

2. Process according to claim 1, characterized in that the biologically active yeast is consisting of compressed yeast or active dried yeast.

3. Process according to claim 1, characterized in that yeast preparations, derived from cultivated strains belonging to the genus Saccharomyces, are used.

4. Process according to claim 1, characterized in that yeast preparations derived from cultivated strains belonging to Torulopsis, are used.

5. Process according to claim 3, characterized in that yeast preparations are used, derived from a strain belonging to a member selected from the group consisting of Saccharomyces cerevisiae, Saccharomyces carlbergensis, Saccharomyces beticus, Saccharomyces rosei, Saccharomyces cheresiensis, Saccharomyces fermenti and Saccharomyces pasteurianus.

6. Process according claim 5, characterized in that yeast preparation are used derived from a Saccharomyces cerevisiae strain.

7. Process according to claims 1-6, characterized in that the treatment of fuels and more particularly petrol will take place during 5-20 days at room temperature.

8. Process according to claims 1-6, characterized in that the treatment of petrol is carried out with 0.01-0.3 kg of yeast, calculated on dry matter pro kg. of fuel.

9. Process according to claim 8, characterized in that the treatment of petrol is carried out with 0.05-0.15 kg. of yeast calculated on dry matter pro kg. of fuel.

10. Fuel treated with biologically active yeast according to the claims 1-9.

11. Petrol treated with biologically active yeast according to the claims 1-9.